Europäisches Patentamt

European Patent Office

Office européen des brevets

(19)

(11) Publication number: **0 189 925**
**A2**

(12) # EUROPEAN PATENT APPLICATION

(21) Application number: 86101227.6

(22) Date of filing: 30.01.86

(51) Int. Cl.⁴: **G 01 N 33/543**
G 01 N 33/53, G 01 N 33/74

(30) Priority: 30.01.85 US 696280
23.01.86 US 820452

(43) Date of publication of application:
06.08.86 Bulletin 86/32

(84) Designated Contracting States:
BE CH DE FR GB IT LI NL SE

(71) Applicant: GENETIC DIAGNOSTICS CORPORATION
160 Community Drive
Great Neck, New York 11021(US)

(72) Inventor: Deutsch, Alice
889 Broadway
New York N.Y. 10003(US)

(72) Inventor: Sheets, Eric J.
240-01 43rrd Avenue
Douglaston N.Y. 11363(US)

(72) Inventor: Rhodes, John
P.O. Box 343
Summit N.J. 07901(US)

(74) Representative: Eggert, Hans-Gunther, Dr.
Räderscheidtstrasse 1
D-5000 Köln 41(DE)

(54) Self-timed immunoassay device.

(57) A process for determining the presence of a particular immunological component A in a test sample, comprising immobilizing at one end of a wick a specimen of said immunological component A or of an immunological component specific to A, passing through said wick said sample and a first reagent comprising an enzyme conjugated to an immunological component B which is either specific to said immunological component A, or immunologically comparable to A, whereby the enzyme-carrying reagent attaches to antigen sites if such sites have not previously been filled by immunological component A in the sample, then passing through the wick by capillarity a second reagent containing a substrate acted upon by the enzyme of the first reagent, and determining the extent of enzymatic reaction on the substrate, the extent of enzymatic reaction indicating the amount of the immunological component in the initial sample. Various alternative processes are provided, including using an immunological reagent with a fluorescent or like label which may compete with the test sample, the wick being assayed for the presence or absence of such label; this eliminates the need for an enzyme and substrate. Kits are also provided.

This is a continuation-in-part of Application Serial No. 696,280, filed January 30, 1985, now pending.

The present invention relates to a simplified process for conducting immunoassays and to kits and components useful in such process.

Heterogeneous assays are usually conducted by contacting a solid phase separator such as plastic with antibody or antigen, labelled, for example, with an enzyme and with a sample to be determined for its antigen content, in either order, followed ultimately by contact with a substrate solution for the enzyme and a reading of the solution or solid separator. Between the individual steps, washing is generally required to eliminate unbound antigens or antibodies which would interfere with the assay.

While this gives satisfactory results, it takes considerable time and operator skill.

It is accordingly an object of the present invention to provide a simplified process for conducting such an assay and a kit suitable for carrying out the process.

It is a further object of the present invention to provide a heterogenous assay process which does not require extensive washings between steps.

These and other objects and advantages are realized in accordance with the present invention pursuant to which there is provided a process for conducting a heterogenous immunoassay comprising wetting one end of a wick with a solution containing a sample to be tested for its antigen content and a first reagent comprising an enzyme conjugated to an antibody which is specific to the antigen to be determined. Either the sample or the first reagent can be added first or preferably, they can be added to one end of the wick at the same time. The other end of the wick carries an immobilized antigen (antigen-wick) which is antigenically similar to the antigen to be determined, i.e., which is identical in antigenic reaction insofar as the instant assay is concerned. After sufficient time for the solution to travel through the wick by capillarity, so as to traverse the wick, a second reagent, a substrate solution for the enzyme, is permitted to wick through the antigen-wick. The substrate solution is acted upon by the enzyme which preceded it in the form of antibody-enzyme and finally a reading is made of the extent of enzyme action, either on the solution which leaves the wick or preferably on the wick itself, e.g., its final color compared with its initial color or its fluorescence.

If the sample contains the antigen, it will combine with the antibody-enzyme while moving along the wick so that when this mixture subsequently wicks through the antigen-wick, there will be no free antibody-enzyme to bind to the antigen on the wick, so it will pass out of the wick. Then when the substrate passes through the wick there will not be any enzyme held on the wick to act upon the substrate so there will be no change in the substrate.

However, if the sample contains no antigen, when the enzyme-labeled antibody reagent contacts the immobilized antigen on the wick, the antigen and antibody will interact thereby securing the enzyme label to the wick. When the substrate solution later passes the secured enzyme, it will be acted upon and there will be a readable change in the substrate, e.g., a change in color or fluorescence. The change can be qualitative, i.e., yes or no as in a pregnancy test, or it can be quantitative to tell how much antigen was in the sample.

A somewhat simplified but less precise test involves eliminating the substrate so that there is no point in having the label be an enzyme. Instead, the label on the reagent is directly readable, e.g., a fluorescent label. If the antigen in the sample binds with all the fluorescent-labeled antibody, then there is no free labeled antibody to attach to antigen immobilized on the wick and the sample antigen will pass through the wick

- 2 -

rather than being held thereby. -3- If one then looks at the wick in a fluorescent light it will not fluoresce. However, if the sample is free of antigen, then the fluorescent-labeled antibody of the reagent will bind to antigen on the wick and the wick will fluoresce.

In the foregoing descriptions, antigen or the antibody may be immobilized on the wick and the first reagent (perhaps the only reagent) may be immunologically the same or complementary to the immunological component immobilized on the wick. The reactions producing or failing to produce a detectable change in the wick or last effluent from the wick can be direct or competitive, relative to the material immobilized on the wick.

The wick can be composed of any capillary active material which, desirably, is inert to the reagents but on which an antigen or antibody can be immobilized in known manner. It can even be composed of different materials in succession, the antigen or antibody being immobilized on one of them. Textile fibrous products have proven quite suitable, preferably in ribbon form. The product can be a woven or knit band but non-wovens have proven especially satisfactory, particularly nylon fiber non-wovens produced by the Pellon Corporation. These may be spun bonded or bonded by heat and pressure and/or extraneous binders.

The ribbon dimensions are not critical, its mass and structure determining the rate of capillary flow. A nylon ribbon about 2 to 15 cm long and about 1 to 5 cm wide, weighing about 30 to 90 grams per square meter is especially suitable.

The portion to which the antigen is immobilized is desirably at least about 3 to 10 cm from the starting end. Stated otherwise, it should take reagent about 5 to 20 minutes to reach the immobilized antigen zone, which then is desirably about 5 to 10 "minutes in length".

The immobilized antigen can be any antigen which will be antigenically similar to the antigen whose

- 3 -

– 4 –

presence in the sample is being tested for. Antigens which can be tested for include dilantin, testosterone, progesterone, etc. They can be as found in normal biological fluids, e.g., exudates, saliva, milk, urine, serum, vaginal smears, and the like, suitably diluted if necessary so as to be transportable by capillarity.

The antigen or antibody can be immobilized on the wick by immersion following chemical reactions to bind protein to the wick as disclosed in Application Serial No. 601,142, filed April 16, 1984, now pending. Thus, for example, the antibody-containing liquid can also contain a bifunctional reagent such as glutaraldehyde or carbodiimide which can be "triggered" by heat to cross-link protein to the wick covalently during or after immersion.

In accordance with other aspects of the invention, kits are provided for carrying out the foregoing processes.

In their simplest form there is a vessel with one or more chambers each containing a reagent liquid, with the wick inside the vessel. The operator starts the process by introducing a sample and optionally a reagent. At some time thereafter a second reagent may be introduced and, with no washing of materials or other steps, the test can eventually be read visually. In a preferred embodiment, all the steps are performed initially and after a predetermined minimum amount of time (during which the operator can do other unrelated things) the operator reads the result.

This aspect of the invention will be described with reference to the accompanying drawings, wherein:

Fig. 1 is a schematic perspective view of one kit for carrying out a process in accordance with the invention;

Fig. 2 is a similar view of another kit;

Fig. 3 is a similar view of yet another kit;

Fig. 4 is a side elevation of yet another kit; and

-5-

Fig. 5 is a top plan view of the kit of Fig. 4.

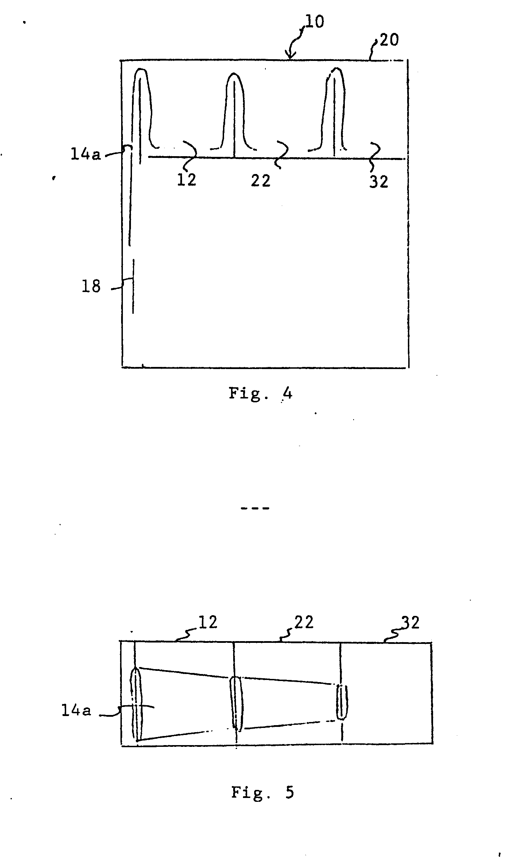

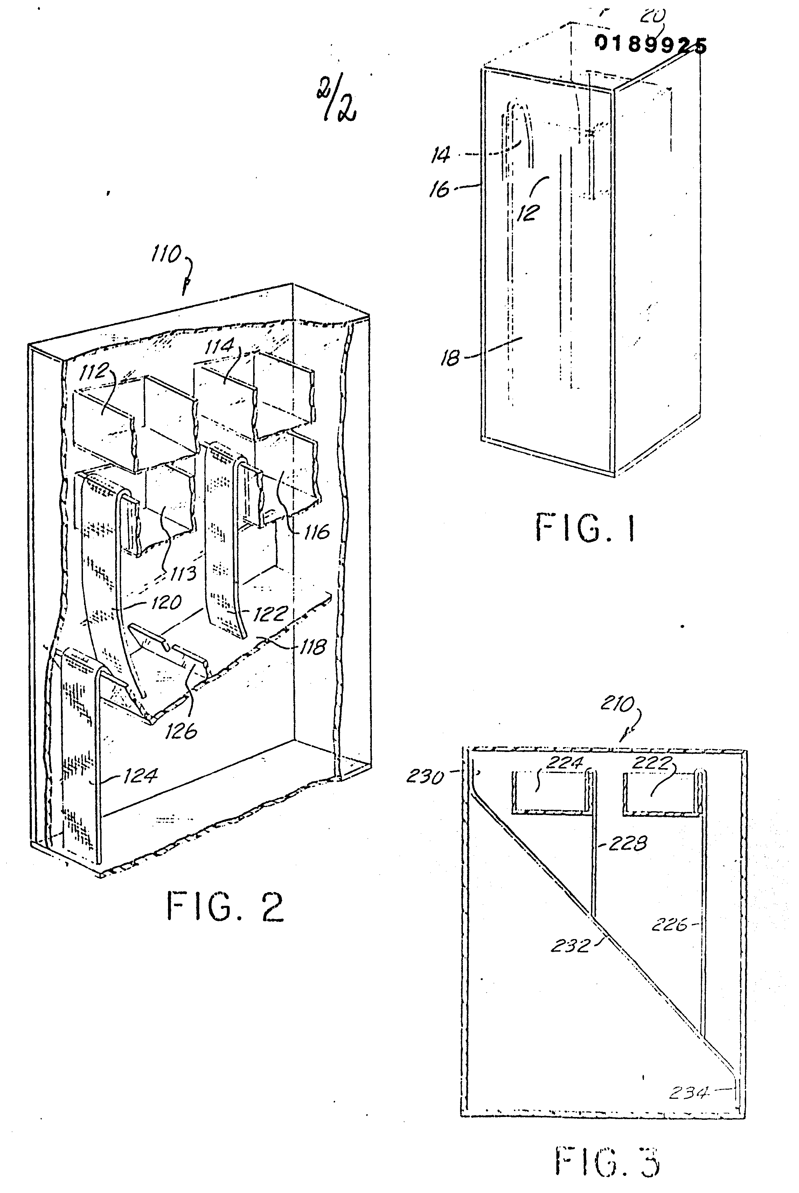

Referring now more particularly to the drawings, in Fig. 1 there is shown a sealed transparent plastic vessel 10 having an open-topped container 12 secured in position above the bottom of the vessel. A wick 14 extends from the bottom of container 12 over its open top and then downwardly along the wall 16 of the vessel 10. A second wick 18 directly contacts wick 14 and is also secured to wall 16, extending to the bottom of the vessel. The wick 18 carries immobilized antigen.

To run a test, the operator pierces the top 20 of vessel 12 as with a pipette and introduces the first reagent and the unknown sample into container 12. A predetermined time later the operator introduces the second reagent (if necessary). The liquids are transported by capillary action and then a determination is made on the liquid now at the bottom of vessel 12 or, preferably, one directly sees whether there has or has not been a color change on wick 18.

In Fig. 2 there is shown a kit containing a plurality of wicks so all the steps preceding reading can be carried out at one time.

A vessel 110 has five containers 112, 113, 114, 116 and 118 secured in place above its bottom, as shown. Container 118 has an inclined plane as its floor and is subdivided by a small notched wall. Wicks 120, 122 and 124 are positioned as shown. The wick 124 carries the immobilized antigen. The test is run as follows: The first reagent and unknown are pipetted into container 112 and the second reagent into container 114. Then each container base is ruptured with a sharp instrument at the same time, allowing the solution in each container to fall into 113 and 116, respectively. Wick 120 transfers material from container 113 to 118 from which wick 124 takes it away.

Wick 122 transfers the second reagent from container 116 to a sub-division of container 118 as a delay device.

- 5 -

Because of the notched wall 126 of container 118, after it fills to a certain height (corresponding to a time delay) as more liquid drips into it, liquid overflows wall 126 into the other side of vessel 118 which by this time is substantially empty of liquid from container 112. Thus the second reagent moves from container 118 through wick 124 a predetermined time after the first reagent. Then the test is read as before on wick 124 and/or the liquid at the bottom of vessel 110.

In Fig. 3, a vessel 210 carries containers 222 and 224. Each has its wick 226, 228 but they act independently so as to transfer liquid at predetermined rates to spaced locations along a wick which has a vertical upper portion 230, an inclined portion 232 and a lower vertical portion 234 which carries the immobilized antigen. Thus sample and/or reagent introduced into container 222 at the beginning of the test reaches wick portion 234 a predetermined length of time before liquid introduced at the same time into container 224.

Fig. 4 shows a variant of Fig. 1, wherein the wick 14a is elongated and also extends into containers 22 and 32 located upstream of container 12. Different liquids are in containers 12, 22, and 32, their compositions being such that the solution in container 12 exits faster than the solution in container 22, and the solution in container 22 exits faster than the solution in container 32. The solutions are thus delivered in sequence to the immunospecific solid surface 18.

Advantageously reagent solutions are made to have different densities so that if two different reagent solutions occupy the same container, 12, 22 and/or 32, on their course through the device, they will layer in discrete phases without mixing together and contaminating one another. The density of the reagent solutions is greatest in the last solution to flow and least in the first solution to flow from any one container (or greatest in the first solution and least in the last solution) with according gradations of density in the interval in solutions. The delivery of uncontaminated reagent solutions in proper sequence is thus ensured.

The term "reagent" as employed herein includes reaction terminators as well as wash liquids intended to separate other liquids from one another in traversing the wick.

The invention will be further described in the following examples wherein all parts are by weight unless otherwise described or apparent.

Example 1: Assay to detect the difference between 0 and 10 µg/ml dilantin.

Nylon Pellon 2518 was treated, using a technique described in U.S. Patent Application Serial No. 601,142, filed April 16, 1984, now pending, to covalently couple dilantin-bovine serum albumin (obtained from Immunotech Inc.) to its surface. Two strips of this antigen-coated Pellon were cut out at 3 X 10 cm. Two strips of untreated Pellon, same dimension, were also cut out. A plastic box was used (dimensions = 10.2 X 5.8 X 3.1 $cm^3$) to contain the trial. The Pellon strips were placed in the box as shown in Fig. 1. Each was slightly moistened so that it stayed against the plastic wall of the box. Two smaller plastic boxes were used (dimensions = 2.1 $cm^3$) to contain assay solutions.

The following reagents were used in the assay:

1) Dilantin monoclonal antibody was coupled to alkaline phosphatase (Boeringher Mannheim) using glutaraldehyde (Avrameas et al 1978, Scand. J. Immunol. 8:7). The dilantin antibody-alkaline phosphatase (DAP) was diluted 1:750 with 1% bovine serum albumin in phosphate buffered saline pH 7.2 (BSA/PBS) before using.

2) Dilantin solution at 10 µg/ml in 1% BSA/PBS was used as sample antigen. Control solution was 1% BSA/PBS alone.

3)    A substrate solution of 2.5 mg/ml disodium 5 bromo-4-chloro-3 indolyl-phosphate (BCIP) in 10% diethanolamine pH 9.6 was used for visualization of enzyme activity.

After all parts of the device were set up as in Fig. 1, the assay was run. First, 3.5 ml DAP and 0.5 ml of 10 µg/ml dilantin were added to chamber 12. After these solutions had passed through the device, by wicking through the nonactivated pellon 14 and then into and through the antigen-Pellon 18, 5 ml BCIP were introduced into chamber 12. The amount of color change on the Pellon was noted (Table I). Total assay time = 45 min. The assay was repeated with 0 µg/ml dilantin.

TABLE I

| Amt. of Dilantin in Sample, µg/ml | Color of Antigen Pellon |
|---|---|
| 0 | blue |
| 10 | white |

The results show that the assay method will detect the difference between 0 and 10 µg/ml of dilantin or between 0 and 1.67 µg dilantin.

Example 2:  Assay in a Self-Contained Device for measuring dilantin.

The purpose of this trial was to use a combination of wicks and compartments so as to set up a self-timed assay device. As shown in Fig. 2, a plastic box was used (dimensions = 10.2 X 5.8 X 3.1 cm$^3$) to contain the various components. Four small plastic boxes and a trough were set up in the plastic container. Two nonactivated Pellon strips and one antigen-coated Pellon strip were used. The reagents for the assay were the same as in Example 1.

-9-

To run the assay, dilantin and DAP were added to box 112 then 6 ml BCIP solution was added to 114. Then the foil seals at the bottoms of boxes 112 and 114 were broken with different pipettes allowing the solutions to flow into box 113 and 116, respectively. Then the solutions passed through the device without further manipulations. Dilantin solution began to wick out of box 113 along 120 and drip into the trough 118. As it dripped into the trough it immediately began to wick into the antigen-coated Pellon 124 and finally accumulated as waste in the bottom of the large plastic box 110. It took 20 minutes for the dilantin solution to pass through the device components. At the same time as the dilantin solution was flowing through the device, the BCIP solution was flowing in the following scheme: BCIP wicked out of box 116 into 118 along wick 122. It collected in 118 behind the subdivider 126 until overflowing. Then BCIP filled up the rest of trough 118. The device was set up such that BCIP only flowed into the trough after all dilantin solution had wicked out of it. Then BCIP wicked over and into the antigen-coated pellon 124. The color change on the Pellon was noted. (Table II). Total assay time = 45 minutes. The experiment was repeated with 0 µg/ml dilantin.

TABLE II — 10 — 0189925

| Amt. of Dilantin in Sample, µg/ml | Color of Antigen Pellon |
| --- | --- |
| 0 | blue |
| 10 | white |

Example 3 :

Using a device as in Figs. 4 and 5, the same wick as in Example 1 was arranged in containers 12, 22 and 32. At the beginning of the trial, dilantin and DAP were added to container 12. Simultaneously, 4 ml wash solution (0.1% Triton X-100 and 1.5% sucrose in PBS) was added to container 22. Then immediately 4 ml BCIP solution containing 2.0 M NaCl was quickly added to container 32. The antigen-Pellon 34 was examined after 60 minutes for any color change. The trial was repeated with 0 µg/ml dilantin. The results of the test on the antigen-Pellon are shown in Table III.

TABLE III

| Amt of Dilantin in Sample, µg/ml | Color of Antigen Pellon |
| --- | --- |
| 0 | blue |
| 10 | white |

Example 4:

This differs from Example 3 in that immobilized antibody, rather than immobilized antigen, is used to detect the difference between 0 and 10 µg/ml dilantin.

Nylon Pellon 2518 was treated, as in Example 1, to covalently couple dilantin specific antibody to its surface. Then the antibody-Pellon 18 was placed in the device as shown in Fig. 4.

The following reagents were used in the assay:

1) Dilantin conjugated to alkaline phosphatase

-11-

(Immunotech) diluted 1:150 in 1% BSA/PBS.

2)    Dilantin solution at 10 µg/ml in 1% BSA/PBS as sample antigen.

3)    Wash solution of 0.1% Triton X-100 and 1.5% sucrose in PBS, pH 7.4.

4)    Substrate solution of 2.25 mg BCIP in 2 M NaCl in 10% diethanolamine buffer, pH 9.8

To run the assay, dilantin and dilantin-alkaline phosphatase were added to container 12.  Simultaneously, 4 ml wash solution was added to container 22.  Then immediately 4 ml BCIP solution was added to container 32. The device was the same as that described in Example 3 except that in this example there was antibody immobilized on the Pellon, not antigen.  After 60 minutes, the antibody-Pellon was examined for color change.  The trial was repeated with 0 µg/ml dilantin.

The results are shown in Table IV.

TABLE IV

| Amt. of Dilantin in Sample, µg/ml | Color of Antibody Pellon |
|---|---|
| 0 | white |
| 10 | blue |

Example 5:

The purpose of this assay was to use a continuous tapered wick in a device and solutions of differing densities to detect the difference between 0 and 20 µg/ml dilantin.

The reagents for the assay were as follows:

1)    Dilantin monoclonal antibody coupled to alkaline phosphatase diluted 1:750 in 1% BSA/PBS(DAP).

2)    Dilantin solution at 20 µg/ml in human urine as

sample antigen. — 12 —

3) Wash solution of 20% sucrose in PBS, pH 7.4.

4) Substrate solution of 2.25 mg BCIP and 40% sucrose in 10% diethanolamine buffer, pH 9.8.

After all parts of the device were assembled as in Fig. 4, the assay was run. First, 0.8 ml dilantin and 0.2 ml DAP were added to container 12. Simultaneously, 1 ml wash solution was added to container 22. Then immediately 1 ml BCIP solution was quickly added to container 32. The antigen-Pellon 18 was examined after 60 minutes for any color change. The trial was repeated with 0 µg/ml dilantin. The results of the color change on the antigen-Pellon are shown in Table V.

TABLE V

| Amt. of Dilantin in Sample, µg/ml | Color of Antigen Pellon |
| --- | --- |
| 0 | blue |
| 20 | white |

It will be understood that the specification and examples are illustrative but not limitative of the present invention and that other embodiments within the spirit and scope of the invention will suggest themselves to those skilled in the art.

C l a i m s

1. A kit comprising

   a) a vessel,

   b) a capillary-active wick extending from the interior of said vessel so as to wick a liquid out of the interior of said vessel, at least a portion of the wick carrying an immobilized immunological component selected from the group consisting of an (i) antigen and an (ii) antibody,

   c) a first reagent comprising an enzyme conjugated to an immunological component selected from the group consisting of an (i) antibody and an (ii) antigen specific to (i) or (ii) respectively of (b), and

   d) a substrate for the enzyme of (c).

2. A kit according to claim 1, including at least one additional vessel, and a second reagent in said additional vessel, said wick extending successively into the interiors of said first vessel and said additional vessel, whereby the reagents within said vessels successively pass through said wick by capillarity.

3. A kit according to claim 2, wherein the reagent in at least one of the vessels comprises at least two liquids of different densities.

4. A kit comprising

   a) a vessel,

   b) a capillary-active wick extending from the interior of said vessel so as to wick a liquid out of the interior of said vessel, at least a portion of the wick carrying in immobilized immunological component selected from the group consisting of an (i) antigen and (ii9 antibody, and

c)    a reagent comprising a fluorescent label tagged to an immunological component selected from the group consisting of an (i) antibody and (ii) antigen specific to (i) or (ii) respectively of (b).

5.    A kit according to claim 4, including at least one additional vessel, and a second reagent in said additional vessel, said wick extending successively into the interiors of said first vessel and said additional vessel, whereby the reagents within said vessels successively pass through said wick by capillarity.

6.    A kit according to claim 5, wherein the reagent in at least one of the vessels comprises at least two liquids of different densities.

7.    A kit comprising
    a)    two reagents of which (i) is an immunological component selected from the group consisting of an antigen and an antibody coupled to an enzyme, and (ii) is a substrate for the enzyme of (i),
    b)    a capillary active wick carrying an immobilized immunological component selected from the group consisting of an antibody and an antigen specific respectively to the antigen or antibody of (a), and
    c)    means for delivering the reagents to the immobilized immunological component of the capillary active wick at different times.

8.    A capillary-active wick linked to an antigen or antibody.

9.    A wick accordung to claim 8, wherein the linkage is through a protein which is complexed to the antigen or antibody.

10. A process for determining the presence of a particular immunological component A in a test sample, comprising immobilizing at one end of a wick a specimen of said immunological component A or of an immunological component specific to A, passing through said wick said sample and a first reagent comprising an enzyme conjugated to an immunological component B which is either specific to said immunological component A, or immunologically comparable to A, whereby the enzyme-carrying reagent attaches to antigen sites if such sites have not previously been filled by immunological component A in the sample, then passing through the wick by capillarity a second reagent containing a substrate acted upon by the enzyme of the first reagent, and determining the extent of enzymatic reaction on the substrate, the extent of enzymatic reaction indicating the amount of the immunological component in the initial sample.

11. A process in accordance with claim 10, wherein the immunological component of A is an antibody and the immunological component of B is an antibody both of which are specific to an antigen.

12. A process in accordance with claim 10, wherein the immunological component A is an antigen and immunological component B is an antibody specific to said antigen.

13. A process in accordance with claim 10, wherein the immunological component A is progesterone and the immunological component B is an antibody specific to progesterone.

14. A process in accordance with claim 8, wherein the immunological component A is an antibody and

-16-

immunological component B is an antigen to which said antibody is specific.

15.    A process in accordance with claim 10, wherein the enzyme is alkaline phosphatase.

16.    A process for determining the presence of a particular immunological component A in a test sample, comprising immobilizing at one end of a wick a specimen of said immunological component A or of an immunological component specific to A, passing through said wick said sample and a reagent comprising an immunological component carrying a label, the immunological component of said reagent being specific to A or immunologically comparable to A, whereby there are potentially competitive reactions on the end of the wick, and then assaying the wick for the presence or absence of said label.

10

20

14a

12 22 32

18

Fig. 4

- - -

12 22 32

14a

Fig. 5

0189925

FIG. 1

FIG. 2

FIG. 3